# EUROPEAN PATENT APPLICATION

(11) **EP 4 775 125 A1**
(43) Date of publication of application: **15.07.2026**
(21) Application number: 24862089.0
(22) Date of filing: 06.09.2024
(51) Int. Cl.: A61B 1/012, A61M 25/10, A61B 1/018, A61B 1/04, A61M 29/02, A61M 31/00, A61B 5/01, A61B 5/22, A61B 17/221

(54) **MEDICAL ENDOSCOPE, SAFE-INSERTION-TYPE ENDOSCOPE DEVICE AND SURGICAL SYSTEM**

(30) Priority: 10.09.2023 CN 202311162691
(71) Applicant: Anhui Happiness Workshop Medical Instruments Co., Ltd, Bengbu, Anhui 233090 (CN)
(72) Inventor: DONG, Dongsheng, Beijing 101116 (CN)
(74) Representative: Eisenführ Speiser
(86) International application number: PCT/CN2024/117501
(87) International publication number: WO 2025/051251

(57) **Abstract**

The present patent relates to the field of medical instruments. Provided are a medical endoscope, a safe-insertion-type endoscope device and a surgical system. In the medical endoscope, the head top end of an endoscope main body is covered with an expandable balloon, the balloon comprises a balloon connecting portion and a balloon free portion, the inner surface area of the balloon free portion is connected to a hollow balloon extension pipe, and the balloon extension pipe is provided with a balloon extension pipe inner opening and a balloon extension pipe outer opening; the balloon extension pipe can extend into an endoscope operation channel directly or under the assistance of a guidewire, and the tip of the balloon extension pipe extends out of an endoscope operation channel outer opening; and the junction of the balloon free portion and the balloon connecting portion extends outward to form a flexible balloon shielding portion. When the endoscope main body is blocked when advancing into a natural orifice of a living body, the inner cavity of the balloon is filled with fluid through a perfusion gap to expand the balloon free portion, so as to radially and flexibly expand the tissue at the narrow part of the natural orifice, thereby making it convenient for the endoscope main body to advance in the expanded natural orifice to significantly reduce shearing injuries to the mucosa during the endoscope insertion process.

## Description

### TECHNICAL FIELD

The present disclosure relates to a medical endoscope, a safe-insertion-type endoscope apparatus, and a surgical system belonging to the technical field of medical device products.

### BACKGROUND

Existing medical endoscopes are classified into a rigid endoscope having an elongated body that cannot be bent or is not easily bent, a flexible endoscope having a body capable of being bent easily, and a combined endoscope in which a rigid endoscope is sleeved outside a flexible endoscope. The rigid endoscope, due to its high strength, can be easily inserted into passages of a living body such as natural orifices or pathological sinus tracts. Although the flexible endoscope has the disadvantage of difficult placement, it is more elongated and flexible, enabling prolonged contact with the natural orifices during operation and causing less tissue trauma than the rigid endoscope. For example, in transurethral laser lithotripsy for kidney stones, the typical procedure is as follows. 1. A rigid endoscope enters bladder and ureter via urethra. 2. A guidewire is then inserted into renal pelvis via a working channel of the rigid endoscope. 3. The rigid endoscope is withdrawn, leaving merely the guidewire in the urinary tract. 4. A ureteral sheath and a dilator located inside the sheath are then placed over the guidewire. 5. The dilator is withdrawn from the ureteral sheath. 6. The flexible endoscope is then inserted into the renal pelvis through the ureteral sheath, where functions such as laser lithotripsy, cooling irrigation, and stone retrieval are conducted via a working channel with an opening at a head portion of the flexible endoscope. The placement of the flexible endoscope involves multiple steps, leading to cumbersome operation. In addition, regardless of being a rigid or flexible endoscope, when advancing inside the natural orifice of the living body, a rigid head edge of an endoscope assembly is in continuous dynamic contact with mucosa of the natural orifice, making shear injury unavoidable. Such shear injuries become more severe when encountering a narrow portion in the natural orifice, and may even result in perforation of the natural orifice. Safety protection measures during endoscope placement in the prior art are often inadequate or even nonexistent, so that secondary infection and functional impairment are prone to occur after the mucosa of the natural orifice suffers from shear injury.

The present disclosure provides a medical endoscope, a safe-insertion-type endoscope apparatus, and a surgical system, where an inflatable balloon structure is disposed at a head portion of the endoscope, providing integrated endoscope-sheath head protection and enabling simultaneous placement of the endoscope and the sheath. A flexible balloon shielding portion can prevent delicate mucosa of the natural orifice from shear injury caused by a rigid head edge of a sheath main body throughout the entire process. When the apparatus encounters resistance during advancement in the natural orifice, a fluid can be infused into an inner cavity of the inflatable balloon from outside the body to cause the balloon to inflate, thereby flexibly dilating the natural orifice radially, and upon deflation, the balloon is restored its original shape, at this time, the dilated natural orifice has not yet recovered to an original shape, allowing for subsequent advancement of the apparatus with minimal resistance. This feature is particularly valuable when encountering a narrow portion in the natural orifice. The present disclosure eliminates cumbersome steps while fully ensuring the safety of the natural orifice of the living body, and improves operational efficiency.

### SUMMARY

An objective of the present disclosure is implemented as follows.

A medical endoscope is provided, where an optical component is disposed on an elongated endoscope main body, the endoscope main body is provided with a working channel extending through an entire length of the endoscope main body, and the working channel is provided with a working channel internal opening configured to enter a human body during use and a working channel external opening configured to be located outside the human body during use; a head tip of the endoscope main body is covered with an inflatable balloon, the balloon is composed of a balloon connecting portion and a balloon free portion, the balloon connecting portion is cylindrical and connected to an outer surface of a head portion of the endoscope main body, the balloon free portion is an inflatable part of the balloon, and a gap between the balloon free portion and the head tip of the endoscope main body forms an inner cavity of the balloon; a maximum outer diameter of the balloon free portion after inflation is at least larger than an outer diameter of the head portion of the endoscope main body; the balloon free portion is transparent, or at least a region of the balloon free portion corresponding to the optical assembly at the head tip of the endoscope main body is transparent; and a flexible balloon shielding portion extends outward from a junction of the balloon free portion and the balloon connecting portion.

A more prominent structure is that a hollow balloon extension tube is connected to an inner surface region of the balloon free portion, the balloon extension tube is provided with a balloon extension tube internal opening and a balloon extension tube external opening; the balloon extension tube can be inserted into the working channel of the endoscope either directly or with assistance of a guidewire, and a tip of the balloon extension tube passes out of the external opening of the working channel of the endoscope; an external fluid can enter the inner cavity of the balloon via a perfusion gap between an outer surface of the balloon extension tube and an inner surface of the working channel to inflate the balloon free portion.

The inner cavity of the balloon extension tube allows passage of the guidewire, serving as a passage for guidewire placement and providing assistance for advancement along the guidewire, but also enables injection of drugs, including lubricating, diagnostic, and therapeutic drugs, into a natural orifice or a pathological sinus tract, and further allows injection of dilating, diagnostic, and therapeutic gases. The balloon extension tube moves in the working channel of the endoscope, and can pull the balloon free portion in an inflated state to reset, control an inflation degree, and adjust a shape of the balloon free portion in the inflated state, thereby meeting an individual operation requirement of a patient or a physician; and in the presence of the balloon extension tube, the external fluid for filling the balloon free portion needs to be injected into the inner cavity of the balloon through a perfusion gap between an outer surface of the balloon extension tube and an inner surface of the working channel of the endoscope.

The guidewire is inserted into the balloon extension tube, so that the medical endoscope can advance under the guidance of the guidewire, or the balloon free portion can perform advancing inflation along the guidewire when filled, thereby dilating a longer natural orifice. To prevent the balloon from gripping the guidewire therein during inflation, which may hinder advancing inflation of the balloon free portion along the guidewire to dilate the natural orifice, the inner cavity of the balloon extension tube and a region of the internal opening of the balloon extension tube are preferably in non-interference contact with the guidewire inserted into the balloon extension tube; a reinforcing structure is disposed at a periphery of the internal opening of the balloon extension tube, including an annular thick-walled reinforcing rib protruding around the internal opening of the balloon extension tube, and a hollow upper thickened section and a hollow lower thickened section which are integrally connected to the internal opening of the balloon extension tube. The guidewire passes through the thickened section, such that the balloon free portion is prevented from being gripped by a potential inward collapse in the region of the internal opening of the balloon extension tube during inflation, thereby ensuring that the balloon free portion can perform advancing inflation along the guidewire when filled.

The balloon is preferably made of a compliant material and may extend forward while flexibly dilating the natural orifice radially. The balloon connection portion is cylindrical and connected to an outer surface of the head portion of the endoscope main body, which is an easy-to-separate connection, such that after a dilation task is completed, the balloon can be removed to exposure the working channel, and other subsequent medical procedures can be performed via the working channel.

The medical endoscope includes a flexible endoscope with a bendable endoscope main body and a rigid endoscope with a non-bendable endoscope main body, and includes both an electronic endoscope and an optical endoscope.

Preferably, a tapered protrusion convex toward a distal end is disposed on the balloon free portion at a periphery of the internal opening of the balloon extension tube. The flexible tapered protrusion helps the endoscope advance in the natural orifice. After the balloon is inflated, the tapered protrusion is pushed to advance further to synchronously dilate the natural orifice further ahead, thereby achieving an extended dilation effect.

A solution capable of increasing the volume of the balloon after inflation is that a hollow papillary protrusion is disposed at a periphery of the internal opening of the balloon extension tube on the balloon free portion, and/or one or more annular folds are disposed at the balloon free portion. The papillary protrusion is conducive to elongational expansion of the balloon free portion after filling; the annular folds can significantly increase surface area of the balloon free portion without changing planar projection area of the balloon free portion at the head tip of the endoscope main body, thereby increasing the volume of the inner cavity of the balloon after inflation. This configuration facilitates axial, elongational expansion of the balloon free portion along the natural body cavity when filled, thereby enabling dilation of a longer natural orifice.

To ensure stable and repeatable inflation of the balloon, a wall thickness and/or strength of the balloon connecting portion of the balloon is greater than that of the balloon free portion, thereby preventing the balloon connecting portion from being pulled and detached from the head portion of the endoscope main body when the balloon free portion is inflated.

For ease of operation of an operator, a hollow elastic motive balloon is connected to the external opening of the working channel; the elastic motive balloon is compressed to fill a fluid therein into the inner cavity of the balloon to inflate the balloon free portion; and after compression is released, the fluid in the inner cavity of the balloon flows back into an inner cavity of the elastic motive balloon. Typically, it is sufficient to utilize gas within the inner cavity of the elastic motive balloon.

The present disclosure further provides a safe-insertion-type endoscope apparatus, including a sheath and the medical endoscope described above, where an elongated sheath main body is sleeved outside an endoscope main body, the sheath main body is provided with a sheath main body internal opening configured to enter a human body during use and a sheath main body external opening configured to be located outside the human body during use. To prevent mucosa of a natural orifice from being sheared by a sharp edge at an internal opening of an inner cavity of the sheath main body, a flexible balloon shielding portion capable of shielding or covering the outer edge of the internal opening of the inner cavity of the sheath main body extends outward from a junction of a balloon free portion and a balloon connecting portion.

Further, the flexible balloon shielding portion extending outward from the junction of the balloon free portion and the balloon connecting portion is provided with a cylindrical flange, and the cylindrical flange fully is configured to cover the outer edge of the internal opening of the sheath. The outer edge of the internal opening of the thin-walled sheath is relatively sharp and is easy to cause shear injury on the mucosa of the contacted orifice during advancement. The flexible balloon shielding portion, which fully covers the outer edge of the internal opening of the sheath, can eliminate the foregoing risk of shear injury. Combined with the dilatable advancement characteristic of the balloon free portion, a dual protective effect on the mucosa of the natural orifice is achieved.

A fastening mechanism capable of being tightly connected to a tail portion of the endoscope main body is disposed adjacent to a region of the external opening of the sheath main body; during use, a head tip of the endoscope main body protrudes from the internal opening of the sheath main body to expose at least a part of the balloon free portion. The fastening mechanism is then started to tightly connect the sheath main body to the endoscope main body, thereby achieving synchronous advancement and retraction of the endoscope main body and the sheath main body in an integrated sheath-endoscope state. When the endoscope main body during advancement encounters an obstruction in the natural orifice of a living body, a fluid can be infused into an inner cavity of the balloon via a perfusion gap to inflate the balloon free portion, thereby flexibly dilate tissues at a narrow portion of the natural orifice radially and facilitating the advancement of the endoscope main body in the dilated natural orifice. After the apparatus reaches a target position, the fastening mechanism is released to disengage the integrated sheath-endoscope state, and the endoscope body can rotate freely or move forward and backward in the inner cavity of the sheath main body.

A fastening mechanism capable of being tightly connected to a tail portion of the endoscope main body is disposed at the external opening of the sheath main body; and the fastening mechanism can be disposed at the tail portion of the sheath main body, or at the tail portion of the endoscope main body. Preferably, the fastening mechanism is disposed at the tail portion of the sheath main body. The fastening mechanism may be an independent element, which is connected to the tail portion of the endoscope main body and the tail portion of the sheath main body during use. The fastening mechanism not only can enable the integrated movement of the endoscope and the sheath, but also can ensure the stability of relative positions of the balloon, the endoscope, and the sheath.

For ease of operation, a section adjacent to the internal opening of the sheath main body is a bendable section of the sheath, and/or a section of the endoscope main body adjacent to the internal opening of the working channel is a bendable section of the endoscope. The bendable section can prevent the endoscope from forming visual and operational blind spots within renal pelvis and calyces.

A transurethral lithotripsy system includes, in addition to the foregoing safe-insertion-type medical endoscope apparatus, includes at least one of a perfusion apparatus, a lithotripsy laser fiber, a negative pressure suction apparatus, a pressure sensor, a temperature sensor, a stone basket, and a guidewire.

The pressure sensor and the temperature sensor can be disposed adjacent to an internal opening of the sheath main body, or on a head portion of an endoscope main body, thereby facilitating intraoperative monitoring of pressure and temperature within the urinary tract and ensuring surgical safety. To reduce friction with mucosa of a natural orifice and various components, a hydrophilic coating is attached to at least one or more of the following surfaces: an outer surface of a balloon free portion, an outer surface of the endoscope main body, an inner surface of a sheath main body, an outer surface of the sheath main body, an outer surface of a balloon extension tube, and an inner surface of a working channel of the endoscope.

The present disclosure has beneficial effects are follows.
1. During advancement of an endoscope of an apparatus within a natural orifice, a rigid head edge of a sheath main body is protected by a flexible balloon shielding portion throughout the entire process, thereby significantly reducing shear injury to mucosa during endoscope placement.
2. When encountering a narrow portion of the natural orifice during endoscope placement, a balloon at the head portion of the endoscope main body is filled and inflated to dilate the tissues at the narrow portion radially, and then the balloon is deflated to facilitate subsequent passage.
3. The fastening mechanism enables synchronous advancement of the endoscope and the sheath, which reduces the number of operational steps and improves efficiency.
4. On the basis of visualization, the balloon at the head portion moves forward in a dilated manner, so that the use of a guidewire during endoscope placement can be omitted.
5. Under pressure monitoring, the balloon at the head portion is inflated to dilate the natural orifice, making the dilation effect safer and more reliable.
6. When the endoscope is placed into the urethra, the balloon at the head portion of the endoscope main body can slowly dilate a membranous part of the urethra radially, thereby protecting external urethral sphincter and minimizing the occurrence of postoperative urinary incontinence.
7. The ureterovesical junction is flexibly dilated to protect the Waldeyer sheath and minimize the occurrence of postoperative vesicoureteral reflux.
8. During advancement of the endoscope apparatus along the natural orifice, drugs such as lubricants and smooth muscle relaxants, or contrast agents, can be injected into the natural orifice through an inner cavity of a balloon extension tube, thereby ensuring the safety of the operation.

The present disclosure significantly simplifies procedural steps, reduces tissue damage, greatly saves operation time, and reduces treatment costs.

### BRIEF DESCRIPTION OF THE DRAWINGS

The accompanying drawings of the present disclosure are not limited as follows.

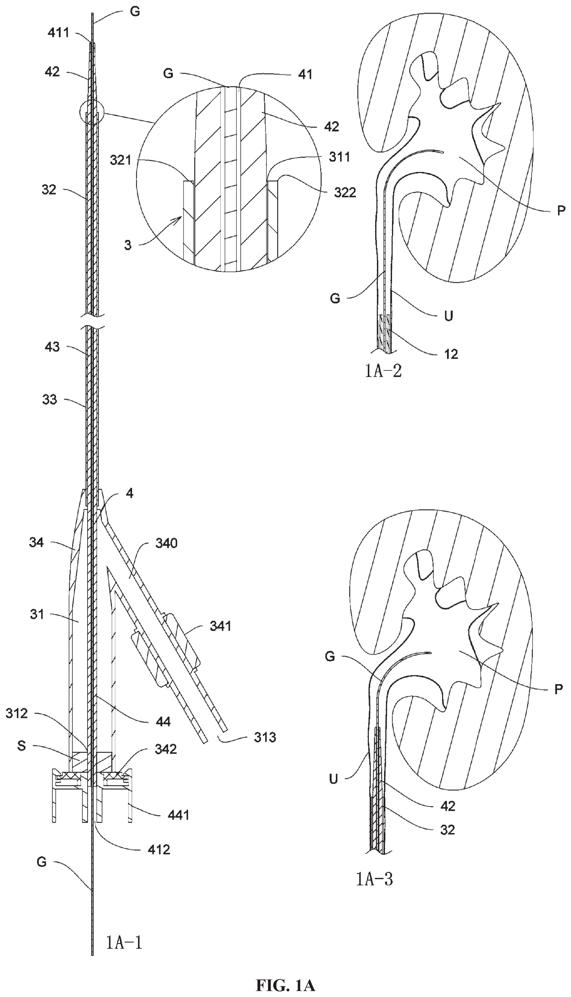

FIG. 1A a diagram showing the use of a sheath, a dilator, and a guidewire in the prior art,
1A-1 shows that a sheath, a dilator, and the guidewire are integrated, 1A-2 shows that a guidewire G is inserted into renal pelvis P using a rigid endoscope, and 1A-3 shows that the dilator is placed in a sheath main body and moves upward along the guidewire G;

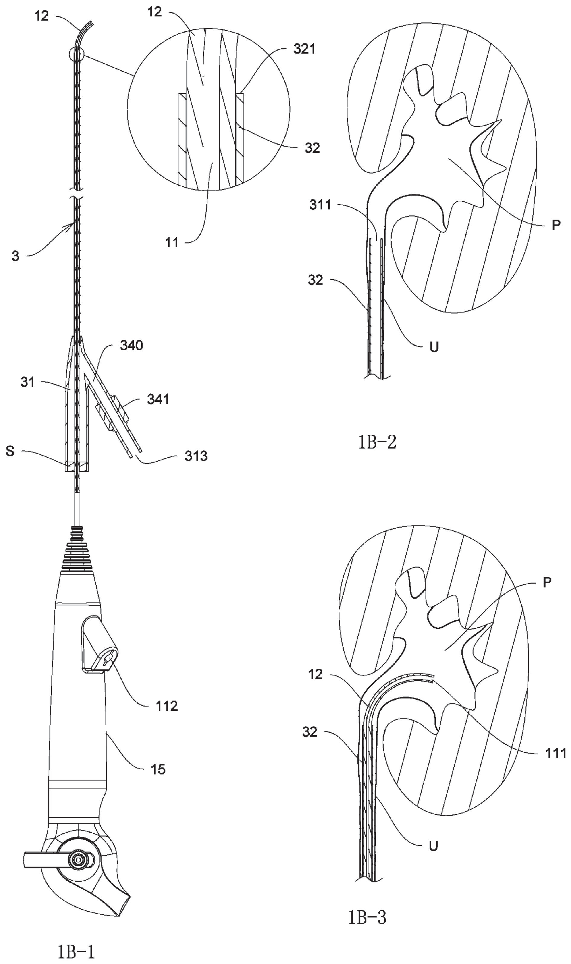

FIG. 1B is a diagram showing entry of an endoscope main body 1 into a sheath main body 3 in the prior art,
1B-1 shows the entry of the endoscope main body 1 into the sheath main body 3, where a bendable head portion 12 of the endoscope main body protrudes from a sheath main body internal opening 321, 1B-2 shows that a head portion 32 of the sheath main body is positioned adjacent to a junction of ureter U and renal pelvis P, and 1B-3 shows that the bendable head portion 12 of the endoscope main body protrudes from the internal opening of the sheath main body and extends into the renal pelvis P;
FIG. 1C is a diagram of an endoscope according to Embodiment 1, in which a head portion 12 of an endoscope main body is covered with an inflatable balloon 2, a balloon extension tube 24 is connected to a balloon free portion 22, the balloon extension tube 24 protrudes from a working channel external opening 112 of the endoscope, an occlusion member 243 can be inserted into a balloon extension tube external opening 242, and a balloon shielding portion 23 is provided with a cylindrical flange 232;
FIG. 1D is a diagram of a sheath main body 3 according to Embodiment 1, which shows a cross-sectional view of a fastening mechanism T at a tail portion 34 of a sheath;
FIG. 1E is a diagram of a combination of an endoscope main body 1 and a sheath main body 3 according to Embodiment 1, which partially shows a cross-sectional view of an elastic dynamic balloon 5 connected to a balloon 2 at a head portion of an endoscope main body and a fastening mechanism T at a tail portion 34 of the sheath that is connected to an endoscope handle 15;
FIG. 1F is a diagram showing a separated state of two clamping arms T1 and a fastening knob T2 of a fastening mechanism T at a tail portion of a sheath main body according to Embodiment 1, wherein a tail portion 14 of an endoscope main body is disposed therein;
FIG. 1G is a perspective diagram of an elastic motive balloon 5 connected to an endoscope handle 15 according to Embodiment 1;
FIG. 1H is a cross-sectional view of a balloon free portion 22 covered at a head portion 12 of an endoscope main body in an uninflated state, where a balloon extension tube 24 is located in a working channel 11 and encounters a narrow portion N in ureter U;
FIG. 1I is a cross-sectional view of a balloon free portion 22 in an inflated state according to Embodiment 1, in which a narrow portion N in ureter U is dilatated radially;
FIG. 1J is a cross-sectional view of a balloon free portion 22 in an inflated state according to Embodiment 1, in which a guidewire G is inserted into an inner cavity 240 of a balloon extension tube, and a balloon free portion 22 is inflated under guidance of the guide wire G;
FIG. 1K is a partially cross-sectional view showing that a clamping arm T1 and a fastening knob T2 are separated after an endoscope and a sheath according to Embodiment 1 are advanced to a junction of ureter U and renal pelvis P, in which a head portion 12 of an endoscope main body enters renal pelvis P from a head portion 32 of a sheath main body;
FIG. 1L is a partially cross-sectional view showing a reverse deformation state of a shielding portion 23 of a balloon free portion after an endoscope main body is withdrawn from an inner cavity 31 of the sheath main body to the outside of the body;
FIG. 2A is a cross-sectional view of a tapered protrusion 228 that is located in a region of an internal opening 241 of a balloon extension tube 24 and extends toward a distal end in a papillary shape;
FIG. 2B is a cross-sectional view showing that a guidewire G is inserted into an inner cavity 240 of a balloon extension tube according to Embodiment 2;
FIG. 3A is a diagram showing an annular fold 227 on a balloon free portion 22, an annular thick-walled reinforcing rib 223 in a region of an internal opening 241 of a balloon extension tube 24, and a downward thickened section 225 according to Embodiment 3;
FIG. 3B is a partially cross-sectional view showing that a guidewire G is inserted into an inner cavity 240 of a balloon extension tube according to Embodiment 3;
FIG. 4A is a diagram of an upward thickened section 224 in a peripheral region of a balloon extension tube internal opening 241 according to Embodiment 4;
FIG. 4B is a partial cross-sectional showing that a guidewire G is inserted into an inner cavity 240 of a balloon extension tube according to Embodiment 4;
FIG. 5A is a cross-sectional view of a hollow papillary protrusion 226 that is located in a peripheral region of an internal opening 241 of a balloon extension tube 24 and protrudes toward a distal end according to Embodiment 5;
FIG. 5B is a cross-sectional view showing that a guidewire G is inserted into an inner cavity 240 of a balloon extension tube according to Embodiment 5, where the guidewire G is non-slotted.

### DETAILED DESCRIPTION OF THE EMBODIMENTS

Embodiments of the present disclosure are not limited as follows.

### Embodiment 1

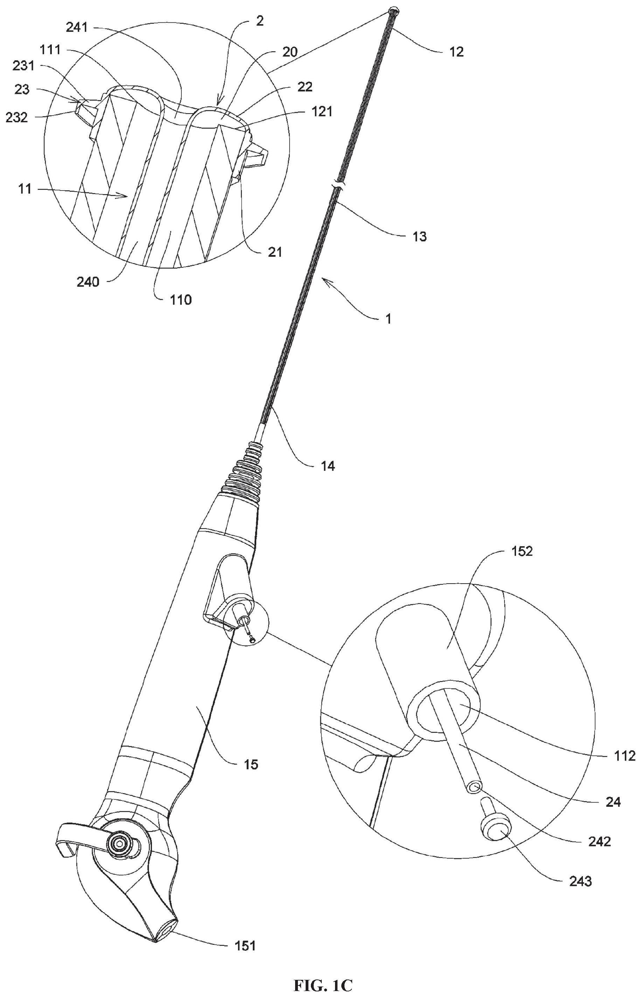

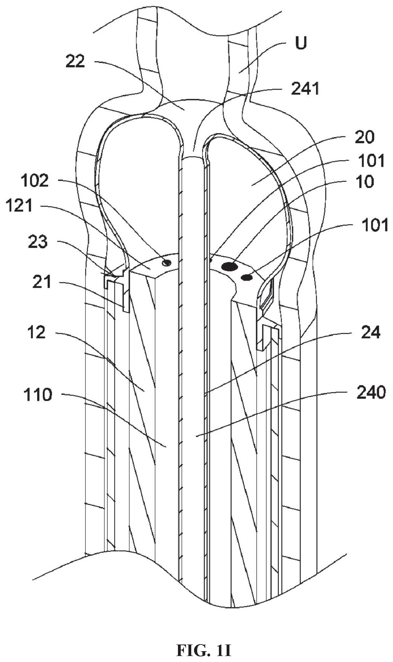

As shown in FIG. 1C, a safe-insertion-type medical endoscope apparatus, includes a bendable elongated endoscope main body 1, which includes an endoscope main body head portion 12, an endoscope main body intermediate portion 13, and an endoscope main body tail portion 14. The endoscope main body 1 is provided with a main channel 11 extending through an entire length of the endoscope main body. The main channel 11 is provided with a main channel internal opening 111 configured to enter a human body during use and a main channel external opening 112 configured to be located outside the human body during use. An operation handle 15 for a user to manipulate is connected to the endoscope main body tail portion 14, and the operation handle 15 is provided with a power interface 151. A head tip 121 of the endoscope main body 1 is covered with an inflatable balloon 2. The balloon 2 is composed of a balloon connecting portion 21 and a balloon free portion 22. The balloon connecting portion 21 is cylindrical and connected to an outer surface of the endoscope main body head portion 12. The balloon free portion 22 is an inflatable part of the balloon 2, and a gap between the balloon free portion 22 and the head tip 121 of the endoscope main body 1 forms an inner cavity 23 of the balloon. A maximum outer diameter of the balloon free portion 22 after inflation is at least larger than an outer diameter of a head 12 tip region of the endoscope main body 1. The balloon free portion 22 is transparent, or at least a region of the balloon free portion corresponding to an optical assembly (as shown in FIG. 1I) of the head tip 121 of the endoscope main body 1 is transparent. The inner cavity 20 of the balloon is in communication with a working channel 11. A hollow balloon extension tube 24 is connected to an inner side of the balloon free portion 22. Most of the balloon extension tube 24 is located within the working channel 11. An inner cavity 240 of the balloon extension tube is provided with two openings, that is, a balloon extension tube internal opening 241 and a balloon extension tube external opening 242. In this embodiment, a proximal portion of the balloon extension tube 24 extends out from an external opening 112 of the working channel of the endoscope on a handle extension section 152, and the external opening 242 of the balloon extension tube can be occluded by an occlusion member 243.

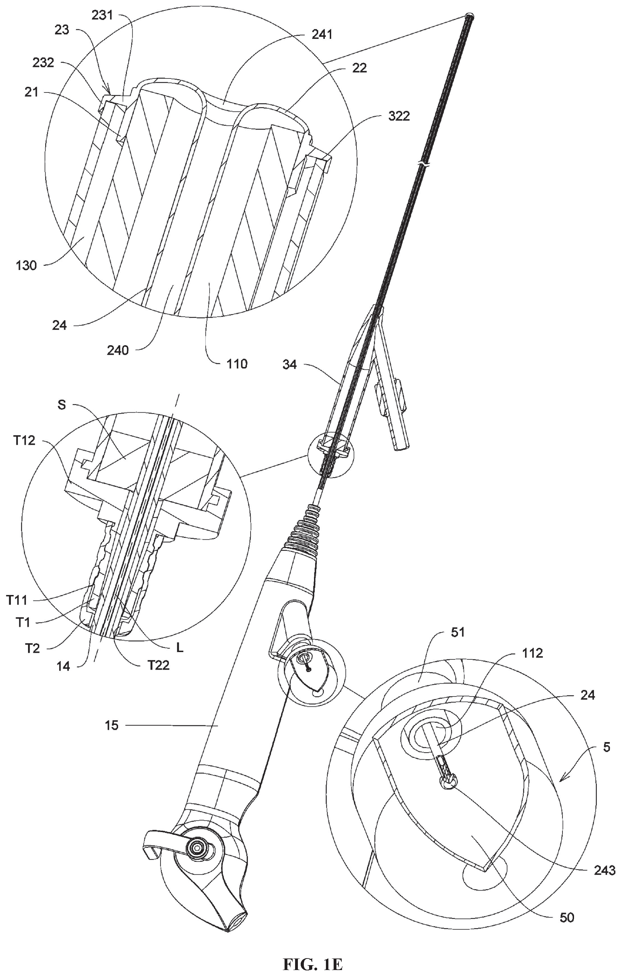

As an outer edge 322 (FIG. 1A-1) of an internal opening of a thin-walled sheath main body 3 is relatively sharp, it is highly likely to cause shear injury to mucosa in a contacted natural orifice during advancement. To protect the mucosa in the natural orifice, a flexible balloon shielding portion 23 capable of shielding or covering the outer edge 322 of the internal opening of the sheath main body 3 extends outward from a junction of the balloon free portion 22 and the balloon connecting portion 21. A cylindrical flange 232 is connected to a horizontal wing 231 of the flexible balloon shielding portion 23, and the cylindrical flange 232 can cover the outer edge 322 of the internal opening of the sheath more thoroughly. FIG. 1E shows that the flexible shielding portion 23 shields the outer edge 322 of the internal opening of the sheath main body 3 and occludes an upper opening of an endoscope-sheath gap 130, such that the ureter U mucosa is free from shear injury when the endoscope is advanced within ureter U. Combined with the dilatable advancement characteristic of the balloon free portion 22, a dual protective effect is achieved.

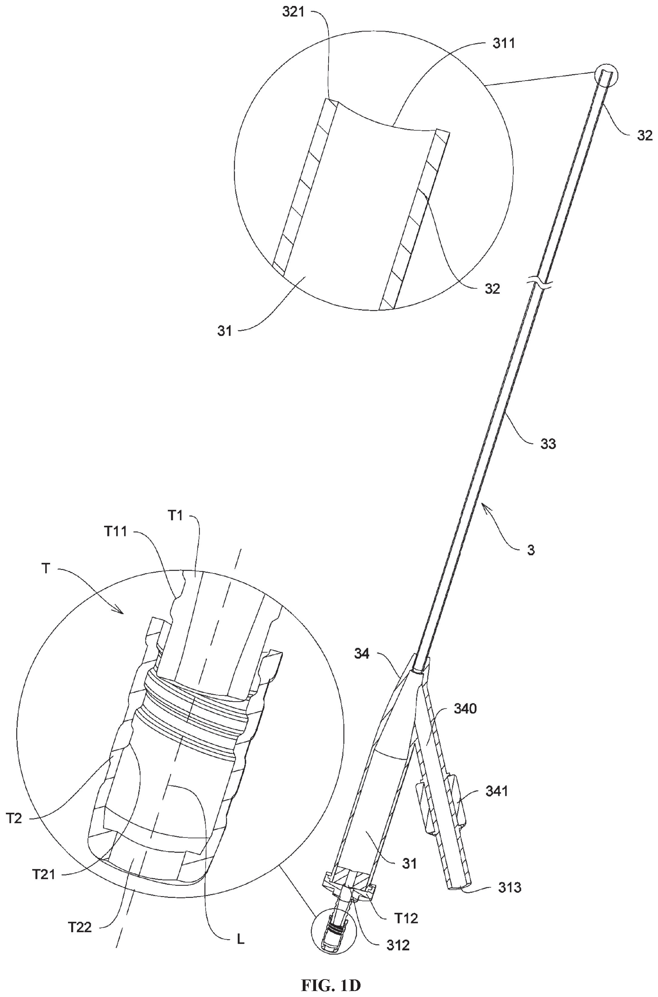

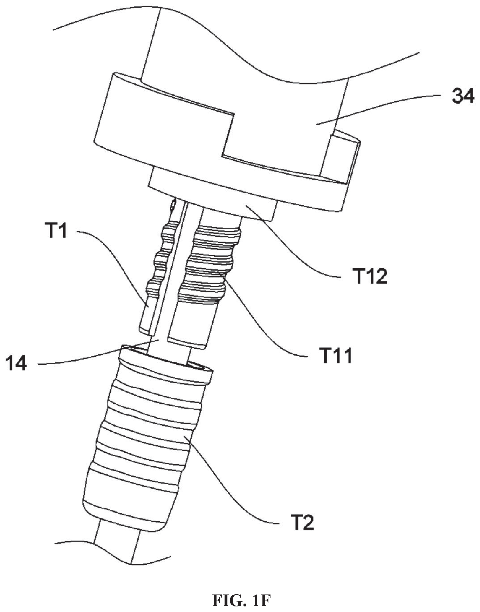

As shown in FIG. 1D, a sheath (referred to equivalently herein as the "sheath main body") is provided for use in conjunction with the endoscope. During use, an elongated sheath main body 3 is sleeved outside over the endoscope main body 1. The sheath main body 3 includes a sheath main body head portion 32, a sheath main body intermediate portion 33, and a sheath main body tail portion 34. The sheath main body 3 is provided with a sheath main body internal opening 311 configured to enter the human body, a sheath main body external opening 312 configured to be located outside the human body during use, and a sheath main body side branch opening 313. An adjustment knob 341 is disposed on a side branch of the sheath main body. When the sheath main body side branch opening is connected to a negative pressure suction apparatus (not shown), the adjustment knob 341 is configured to control whether an inner cavity 340 of the side branch of the sheath is in communication with the outside. A fastening mechanism T capable of being tightly connected to the endoscope main body tail portion 14 is disposed at the external opening 312 of the sheath main body. In this embodiment, the fastening mechanism T is composed of arc-shaped sheet-like clamping arms T1 and a hollow fastening knob T2. The fastening knob T2 is provided with a circular knob external opening T22. Two clamping arms T1 are disposed, and the clamping arms T1 are connected to the sheath main body tail portion 34 via a base T12 thereon. After internal thread T21 of the fastening knob T2 is engaged with external threads T11 of the clamping arms, the two clamping arms T1 move radially toward an axis L to clamp the endoscope body tail portion 14 located inside the clamping arms (as shown in FIG. 1E), so that the endoscope and sheath (herein, "endoscope-sheath" refers to the endoscope and the sheath) can be advanced synchronously. FIG. 1F shows that the two clamping arms T1 are separated from the fastening knob T2, and the clamping on the endoscope main body tail portion 14 by the clamping arms T1 is released. In this case, the endoscope and sheath are no longer tightly coupled, and the endoscope body 1 can move forward and backward and rotate freely in the sheath main body 3.

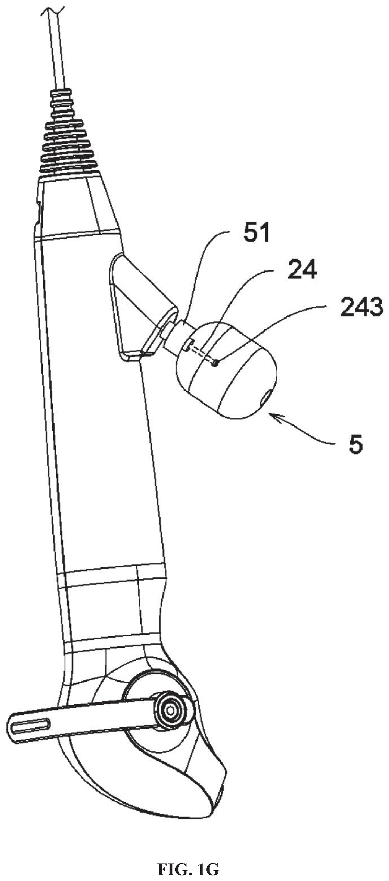

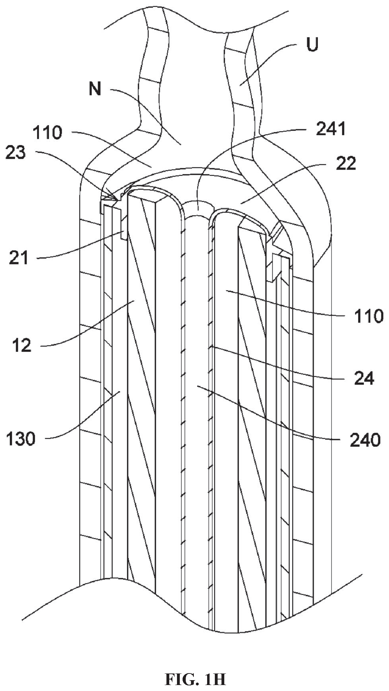

As shown in FIG. 1E and FIG. 1G, a hollow elastic motive balloon 5 is connected to the external opening of the working channel 112 on the endoscope handle. The elastic motive balloon 5 is sleeved outside a handle extension section 152 (as shown in FIG. 1C) via an extension portion 51, so that the external opening 112 of the working channel of the endoscope is in communication with an inner cavity 50 of the elastic motive balloon, and a proximal portion of the balloon extension tube 24 is configured to protrude from the external opening 112 of the working channel of the endoscope and is located in the inner cavity 50 of the elastic motive balloon. During use, the occlusion member 243 is firstly inserted into the external opening 242 of the balloon extension tube for occlusion, the inner cavity 50 of the elastic motive balloon can be filled with gas or liquid, and the inner cavity 50 of the elastic motive balloon is provided with a communicating hole (not shown in the figures) communicating with external environment. When the integrated endoscope and sheath are advanced in the ureter U and encounters a narrow portion N that impedes further advancement (see FIG. 1H), the elastic motive balloon 5 is compressed outside the body to fill the fluid (which is air in this embodiment) therein into the inner cavity 20 of the balloon via a perfusion gap 110. The perfusion gap 110 refers to a gap between an outer surface of the balloon extension tube 24 and an inner surface of the working channel 11 of the endoscope, as shown in FIG. 1I. After the fluid enters the inner cavity 20 of the balloon, the balloon free portion 22 is inflated to flexibly and radially dilate the narrow portion N of the ureter U, which eliminates the obstruction to the advancement at the narrow portion N on the premise of not causing shear injury to the mucosa and facilitates the passage of the endoscope. After the compression is released, the fluid in the inner cavity 20 of the balloon flows back to the inner cavity 50 of the elastic motive balloon, and the shape of the balloon free portion 22 is recovered to its pre-inflation shape. The elastic motive balloon 5 can be repeatedly compressed by or released from fingers, causing the balloon free portion 22 to inflate or deflate synchronously. The self-recovery capability of the elastic motive balloon 5 is sufficiently utilized, making it conveniently to be used. The communication hole 52 on the elastic motive balloon 5 is particularly suitable for gas communication. During compression, a finger presses to seal the communication hole 52, allowing the gas in the elastic motive balloon 5 to fill the inner cavity 20 of the balloon. After the balloon free portion 22 has remained inflated for a certain period, the finger is released to make the elastic motive balloon 5 self-recover and the gas rapidly flow out through the communication hole 52, so that the balloon free portion 22 can be restored more quickly. Certainly, a syringe or other electronically controlled perfusion apparatus, as well as a negative pressure suction apparatus, can be externally connected to control the elastic motive balloon 5.

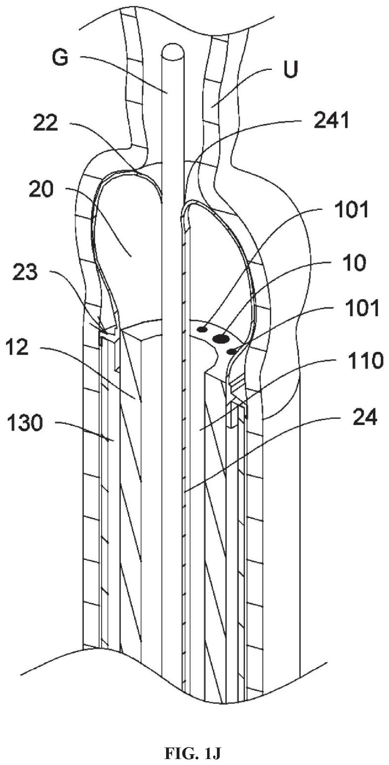

When the apparatus provided by the present disclosure is in dilatable advancement in the natural orifice, in certain cases, if guidance by a guidewire G is required, the guidewire G can be inserted through the inner cavity 240 of the balloon extension tube. FIG. 1J shows that the guidewire G passes out of the internal opening of the balloon extension tube, and the balloon free portion 22 in an inflated state. In this case, the balloon free portion 22 is filled and inflated along the guidewire G, thereby dilating the natural orifice more effectively.

The inner cavity 240 of the balloon extension tube allows the guidewire to pass through, serving as a passage for guidewire placement and providing assistance for advancement along the guidewire. After the occlusion member 243 is removed from the external opening 242 of the balloon extension tube , drugs, including lubricating, diagnostic, and therapeutic agents such as smooth muscle relaxants and contrast agents, can also be injected into the natural orifice via the inner cavity 240 of the balloon extension tube; and dilating, diagnostic, and therapeutic gases can also be injected. The balloon extension tube moves in the working channel of the endoscope, and can pull the balloon free portion in an inflated state to reset, control an expansion degree, and adjust a shape of the balloon free portion in the inflated state, thereby meeting an individual operation requirement of a patient or a physician.

As shown in FIG. 1I, a lens 10 and two optical windows 101 are disposed at the head tip 121 of the endoscope main body 1, along with a combined pressure and temperature sensor 102. The pressure sensor can monitor the pressure of the balloon free portion 22 during inflation, thereby enabling adjustment of a radial dilation force to effectively dilate a narrow natural orifice while ensuring operational safety. The temperature sensor can monitor temperature changes during lithotripsy to prevent thermal injury.

During use, the head tip 121 of the endoscope main body 1 moderately protrudes from the internal opening of the sheath main body 311 to at least expose the balloon free portion 22 to the outside. The fastening mechanism T is started to tightly connect the sheath main body 3 to the endoscope main body 1, thereby achieving synchronous advancement and retraction of the integrated endoscope and sheath. When the endoscope main body 1 encounters an obstruction while advancing in the natural orifice of a living body, the fluid can be infused into the inner cavity 20 of the balloon via the perfusion gap 110 to inflate the balloon, thereby flexibly dilating the narrow portion of the natural orifice radially to facilitate the subsequent advancement of the endoscope main body 1. After the sheath main body 3 reaches a target position, the fastening mechanism T is released, and the endoscope main body 1 can rotate freely or move back and forth in the sheath 3.

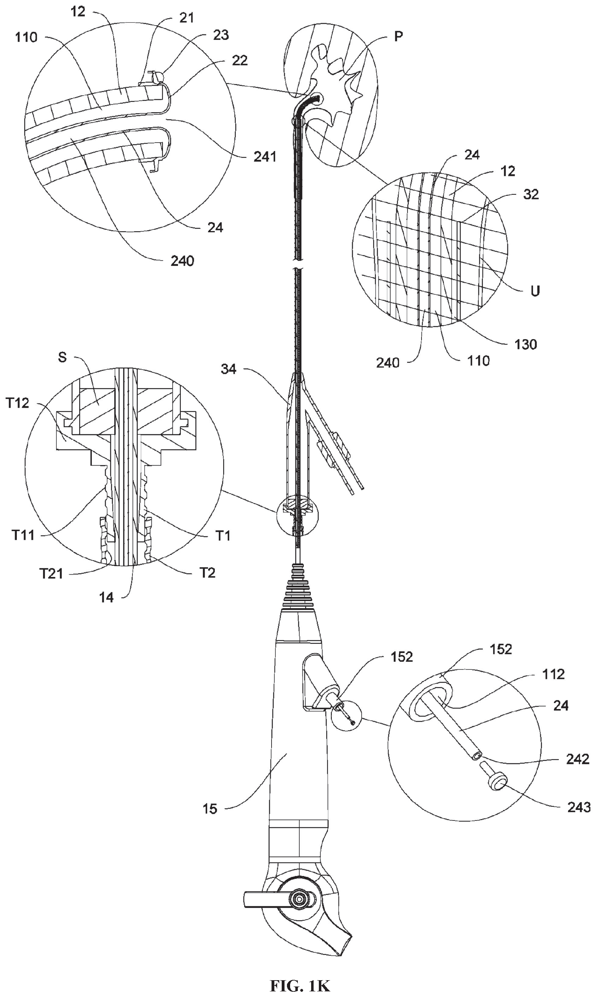

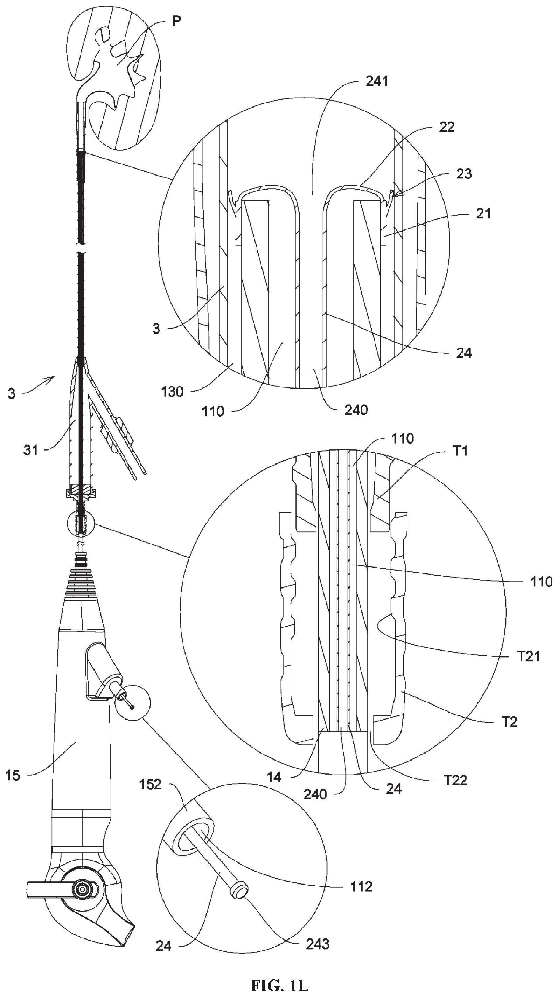

As shown in FIG. 1K, after the integrated endoscope and sheath of the present apparatus is advanced to the target position, the fastening knob T2 is rotated outside the body to disengage the clamping arms T1 from the fastening knob T2. The clamping of the endoscope main body tail portion 14 by the clamping arms T1 is thereby released, allowing the endoscope main body head portion 12 to protrude from the internal opening 311 of the sheath main body to enter the renal pelvis P. As shown in FIG. 1L, the endoscope main body 1 is withdrawn rearward from the inner cavity 31 of the sheath main body. Within an endoscope-sheath gap 130, the shielding portion 23 of the balloon free portion deforms and flips upward. When the head portion 12 of the endoscope main body is withdrawn from the external opening 312 of the sheath, the balloon 2 covering the head portion 12 of the endoscope main body, together with the balloon extension tube 24, is removed through either the upper opening 111 or the lower opening 112 of the working channel 11 to expose the working channel 11. Afterwards, the endoscope main body 1 is then placed into the inner cavity 31 of the sheath main body for subsequent lithotripsy or other medical procedures (not shown).

The balloon connecting portion 21 is cylindrical and is connected to the outer surface of the head portion 12 of the endoscope main body by a connection manner that facilitates separation. This configuration facilitates exposure of the working channel 11 to enable subsequent performance of other medical procedures.

In the present disclosure, regarding the positional descriptions of the various tubular bodies described herein: the "head portion" refers to facing a distal end, which enters the human body during use; the "tail portion" refers to facing a proximal end, which typically remains outside the human body during use; the "inner opening" refers to a distal opening, which is located inside the human body during use; and the "outer opening" refers to a proximal opening, which is located outside the human body during use. "Advancement" refers to a direction toward the distal end, and "retraction" refers to a direction toward the proximal end. "Up" and "front" refer to the direction toward the distal end; "down" and "back" refer to the direction toward the proximal end.

The balloon 2 is preferably made of a compliant material, which may extend forward while flexibly radially dilating the natural orifice.

An endoscope system in the prior art is shown in FIG. 1A and FIG. 1B. A hollow sheath main body 3 includes a sheath main body head portion 32, a sheath main body intermediate portion 33, and a sheath main body tail portion 34. The sheath is provided with a sheath main body internal opening 311 configured to enter the human body during use, a sheath main body external opening 312 configured to be located outside the human body during use, and a sheath main body side branch opening 313. An adjustment knob 341 is disposed on a side branch of the sheath main body. When the sheath main body side branch opening 313 is connected to a negative pressure suction apparatus (not shown), the adjustment knob 341 is configured to control whether an inner cavity 340 of the side branch is in communication with the outside. A hollow elastic sealing plug S is disposed in the external opening 312 of the sheath. A dilator 4 includes a dilator head portion 42, a dilator intermediate portion 43, and a dilator tail portion 44. An inner cavity 41 of the dilator is provided with a dilator internal opening 411 and a dilator external opening 412. The external opening 412 of the dilator protrudes from the tail portion 34 of the sheath main body, a tip of the tail portion 44 of the dilator is located outside the inner cavity 31 of the sheath main body, and the tip of the tail portion 44 of the dilator is provided with a snap-fit body 441. The snap-fit body 441 is in snap-fit with a receiving body 342 at the tip of the tail portion 34 of the sheath main body. After snap-fitting, synchronous advancement and retraction of the sheath and dilator are achieved. The head portion of the dilator 42 is conically tapered and extends from the internal opening 311 of the sheath main body. In the figure, a head tip 321 of the sheath main body 3 and a sharp edge 322 of the internal opening of the sheath are visible. A guidewire G extends through the inner cavity 41 of the dilator and protrudes from the internal opening 411 of the dilator.

In the prior art, the steps for placing an endoscope in the transurethral lithotripsy are as follows. 1. As shown in FIG. 1A-2, the head portion 12 of a rigid endoscope is inserted into the bladder via urethra and then enters the ureter U. 2. A guidewire G is inserted into renal pelvis P through a working channel of the rigid endoscope. 3. The rigid endoscope is withdrawn, leaving merely the guidewire in a urinary tract. 4. As shown in FIG. 1A-3, a ureteral sheath together with a dilator therein is then inserted via the guidewire. 5. The dilator is withdrawn from a ureteral access sheath (not shown). 6. As shown in FIG. 1B-1, a flexible endoscope is then inserted from the inner cavity 31 of the ureteral sheath main body. FIG. 1B-2 shows that the head portion 32 of the sheath main body is located adjacent to a junction of the ureter U and the renal pelvis P, with the internal opening 311 of the sheath main body facing the renal pelvis P. FIG. 1B-3 shows that the head portion 12 of the bendable endoscope main body protrudes from the internal opening 311 of the sheath and extends into the renal pelvis P. The working channel 11 having an opening at the head portion of the flexible endoscope is responsible for functions such as laser lithotripsy, cooling irrigation, and stone retrieval. The procedure for placing the flexible endoscope in the prior art is cumbersome and lacks safety protection.

During advancement of a protective endoscope apparatus of the present disclosure in a natural orifice, a rigid head edge of the sheath main body is protected by the flexible shielding portion throughout the entire process, which significantly reduces mucosal shear injury during endoscope placement. When encountering a narrow portion in the natural orifice during endoscope placement, the balloon at the head portion of the endoscope main body is inflated to radially dilate the narrow portion, after which the balloon is deflated to facilitate subsequent passage. Synchronous advancement of the endoscope and sheath reduces the number of procedural steps and improves efficiency, and both the use of the dilator 4 component and the guidewire G can be omitted. The dilatable advancement of the balloon at the head portion can be achieved under visualization. Under the pressure monitoring, the natural orifice is dilated after the balloon at the head portion is dilated, making the dilation effect more reliably. When the endoscope is placed into the urethra, the balloon at the head portion of the endoscope main body can slowly dilate a membranous part of the urethra radially, thereby protecting external urethral sphincter and minimizing the occurrence of postoperative urinary incontinence. The ureterovesical junction is flexibly dilated to protect Waldeyer sheath and minimize the occurrence of postoperative vesicoureteral reflux. The present disclosure significantly simplifies procedural steps, reduces tissue damage, greatly saves operation time, and reduces treatment costs.

### Embodiment 2

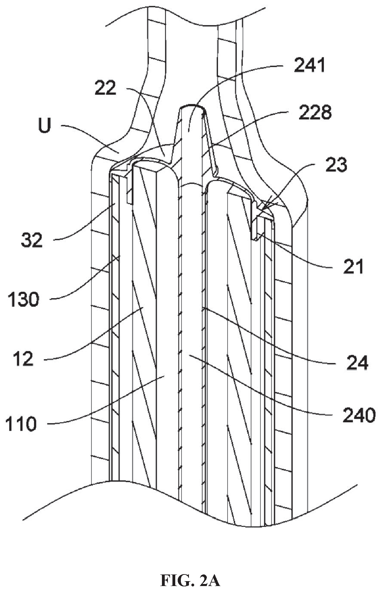

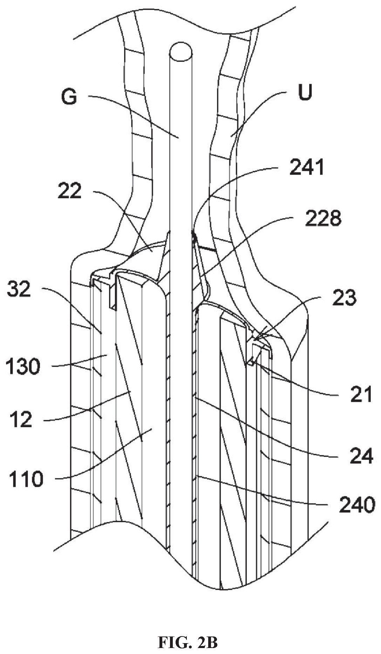

As shown in FIG. 2, in this embodiment, a tapered protrusion 228 convex toward a distal end is disposed on the balloon free portion 22 at the periphery of the internal opening 241 of the balloon extension tube. The flexible tapered protrusion helps the endoscope advance in the natural orifice. After the balloon is inflated, the tapered protrusion is pushed to advance further to synchronously dilate the natural orifice further ahead, thereby achieving an elongational dilation effect.

As shown in FIG. 2B, the guidewire G passes through the inner cavity 240 of the balloon extension tube 24 and the tapered protrusion 228 thereon. The balloon free portion 22 can be inflated under the guidance of the guidewire G, thereby better dilating the natural orifice, particularly a narrow natural orifice.

### Embodiment 3

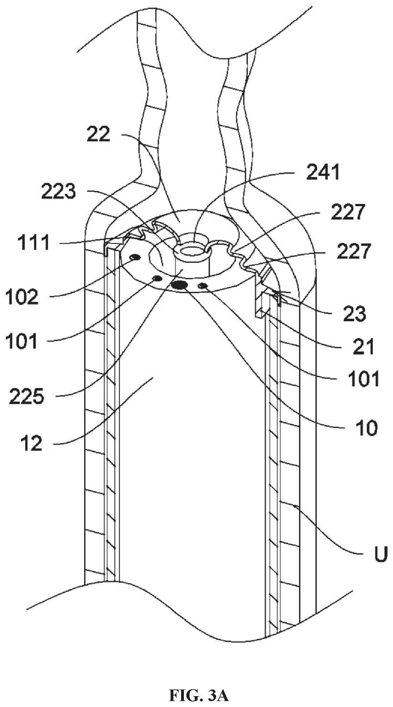

As shown in FIG. 3A, this embodiment differs from Embodiment 2 in that, to enhance the cooperation between the balloon 2 and the guidewire G, that is, to prevent a region of the internal opening 214 of the balloon extension tube from being squeezed inward and clamping onto the guidewire G when the balloon free portion 22 is inflated, which makes the inflated free portion 22 unable to extend along the guidewire, the region of the internal opening 214 of the balloon extension tube is provided with an annular thick-walled reinforcing rib 223 and a downward thickened section 225, such that the balloon free portion 22 does not clamp onto the guidewire G during inflation.

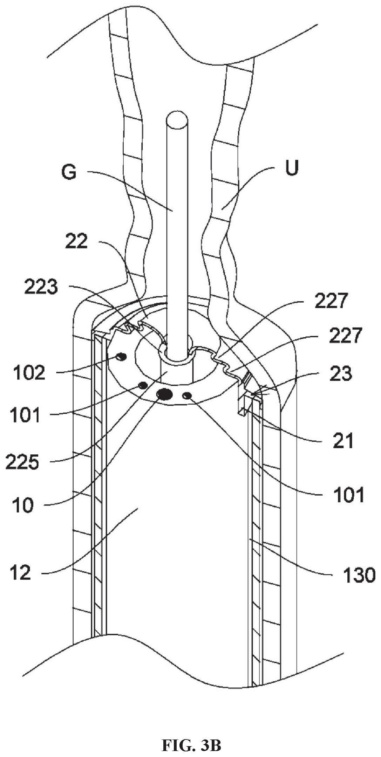

To increase the volume after inflation under a same filling pressure, multiple annular folds 227 capable of increasing area are disposed on the balloon free portion 22. FIG. 3B shows that the guidewire G passes through the inner cavity 240 of the balloon extension tube. The annular folds 227 on the balloon free portion 22 significantly increase the surface area of the balloon free portion 22 without changing projection area of the balloon free portion 22 in a region of the head tip 121 of the endoscope, thereby increasing the volume of the inner cavity of the balloon after inflation. This configuration facilitates axial elongational expansion of the balloon free portion 22 along the natural orifice during filling, enabling dilation of a longer narrow orifice in a single inflation.

### Embodiment 4

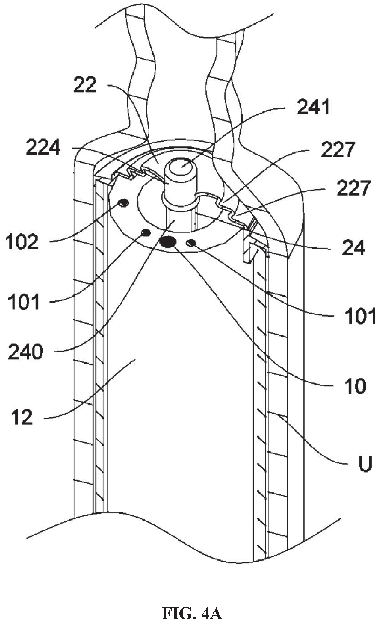

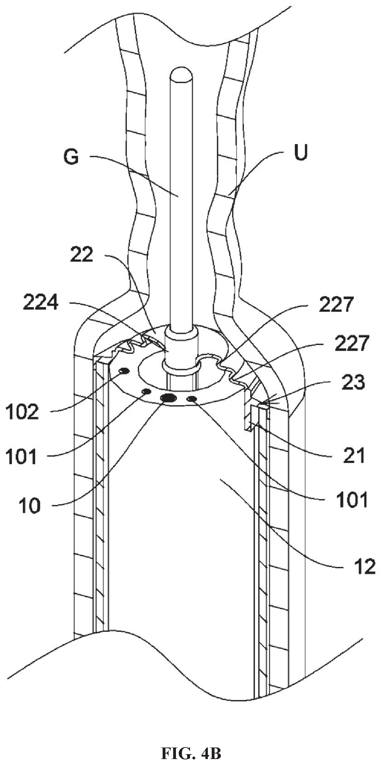

As shown in FIG. 4A and FIG. 4B, the present embodiment differs from Embodiment 3 in that an upward thickened section 224 is disposed in a peripheral region of the internal opening 241 of the balloon extension tube, the purpose of which is still to prevent the balloon free portion 22 from clamping onto the guidewire G during inflation.

### Embodiment 5

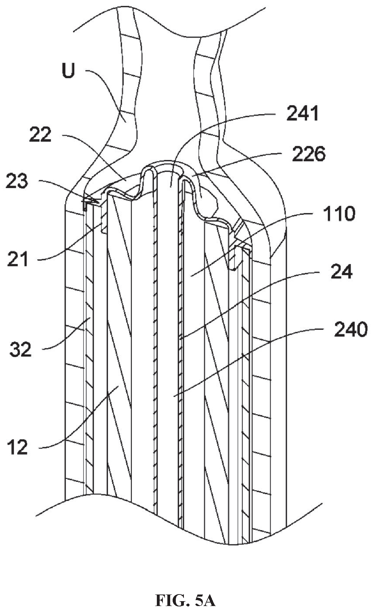

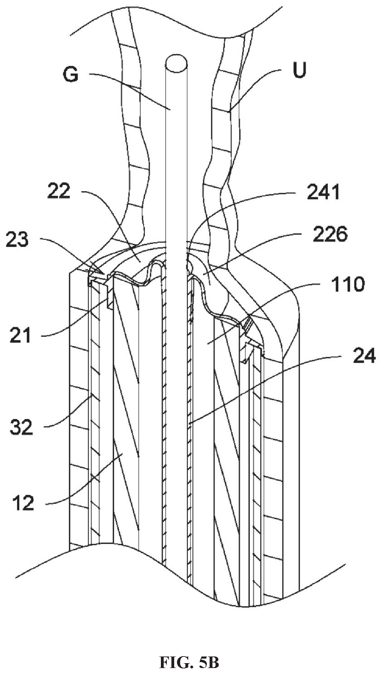

As shown in FIG. 5A, a hollow papillary protrusion 226 convex toward a distal end is disposed in a peripheral region of the internal opening 241 of the balloon extension tube. The hollow papillary protrusion 226 facilitates forward-extending expansion of the balloon free portion 22 during filling, and increases an expansion range and extending an axial distance of expansion. FIG. 5B shows that guidewire G passes through the inner cavity 240 of the balloon extension tube. To ensure that no fluid leaks from the balloon connecting portion 21 when the balloon is inflated, the balloon connecting portion 21 is hermetically connected to the outer surface of the head portion 12 of the endoscope main body, a wall thickness of the balloon connecting portion 21 is greater than that of the balloon free portion 22, and the balloon connecting portion 21 and the outer surface of the head portion 12 of the endoscope main body are preferably in an interference fit. After assembly, the balloon connecting portion 21 is located in the endoscope-sheath gap 130. Although the interference fit is used, the elastic nature of the balloon 2 allows the balloon free portion 22, when subjected to greater external pulling force, to drive the balloon connecting portion 21 to deform upward and finally detach from the head portion 12 of the endoscope main body.

To display a key structure clearly, the change in the wall thickness of the balloon free portion 22 that decreases upon inflation is not shown in the accompanying drawings of the present disclosure.

When the present disclosure is used for lithotripsy, a section of the sheath main body adjacent to the internal opening 311 of the sheath main body is a bendable section of the sheath main body, and/or a section of the endoscope main body adjacent to the internal opening 111 of the working channel is a bendable section of the endoscope (as shown in FIG. 1K). The bendable sections minimize visual and operational blind spots of the endoscope within the renal pelvis and calyces.

When the present disclosure is used in a transurethral lithotripsy system, it further includes at least one of a perfusion apparatus (not shown), a lithotripsy laser fiber (not shown), a negative pressure suction apparatus (not shown), a pressure sensor, a temperature sensor (not shown), a stone basket (not shown), and a guidewire. The pressure sensor and the temperature sensor may be disposed in a region adjacent to the internal opening 311 of the sheath main body or may be disposed on the head portion 12 of the endoscope main body, thereby facilitating intraoperative monitoring of pressure and temperature in the urinary tract and ensuring surgical safety. To reduce friction during placement and operation, a hydrophilic coating is attached to each of the balloon free portion 22, the endoscope main body 1, the sheath main body 3, and the guidewire G.

The safe-insertion-type medical endoscope provided by the present disclosure significantly simplifies the procedural steps, reduces tissue damage, saves surgical time, and lowers treatment costs. The safe-insertion-type medical endoscope is applicable for medical procedures in natural orifices such as cardiovascular system, respiratory tract, ear canal, lacrimal passage, reproductive tract, and digestive tract, as well as in third spaces of the living body such as the thoracic and abdominal cavities, and further applicable to medical procedures in wound tracts of penetrating injuries, pathological sinus tract, and the like.

## Claims

1. A medical endoscope, comprising an elongated endoscope main body (1) on which an optical assembly is disposed, the endoscope main body (1) is provided with a working channel (11) extending through an entire length of the endoscope main body (1), and the working channel (11) is provided with a working channel internal opening (111) configured to enter a human body therewithin during use and a working channel external opening (112) configured to be located outside the human body during use;
wherein, a head tip (121) of the endoscope main body (1) is covered with an inflatable balloon (2), the balloon (2) is composed of a balloon connecting portion (21) and a balloon free portion (22), the balloon connecting portion (21) is cylindrical and connected to an outer surface of a head portion (12) of the endoscope main body (1), the balloon free portion (22) is an inflatable part of the balloon (2), and a gap between the balloon free portion (22) and the head tip (121) of the endoscope main body (1) defines an inner cavity (20) of the balloon; a maximum outer diameter of the balloon free portion (22) after inflation is at least larger than an outer diameter of the head portion (12) of the endoscope main body; the balloon free portion (22) is transparent, or at least a region of the balloon free portion (22) corresponding to the optical assembly at the head tip (121) of the endoscope main body (1) is transparent; a hollow balloon extension tube (24) is connected to an inner surface region of the balloon free portion (22); the balloon extension tube (24) is provided with a balloon extension tube internal opening (241) and a balloon extension tube external opening (242); the balloon extension tube (24) is able to be inserted into the working channel (11) of the endoscope either directly or with assistance of a guidewire (G), and a tip of the balloon extension tube (24) passes out of the external opening (112) of the working channel of the endoscope; a fluid is able to enter the inner cavity (20) of the balloon via a perfusion gap (110) between an outer surface of the balloon extension tube (24) and an inner surface of the working channel (11); a flexible balloon shielding portion (23) extends outward from where the balloon free portion (22) is connected with the balloon connecting portion (21); when advancement of the endoscope main body (1) along a natural orifice of a living body becomes difficult, the fluid is able to be infused into the inner cavity (20) of the balloon via the perfusion gap (110) to inflate the balloon free portion (22), thereby flexibly dilating tissue at a narrow portion of the natural orifice radially to facilitate advancement of the endoscope main body (1) in the dilated natural orifice.

2. The medical endoscope according to claim 1, wherein the balloon free portion (22) is provided with a tapered protrusion (228) located at a peripheral region of the internal opening (241) of the balloon extension tube and convex toward a distal end.

3. The medical endoscope according to claim 1, wherein the balloon free portion (22) is provided with a hollow papillary protrusion (226) located at a peripheral region of the internal opening (241) of the balloon extension tube, and/or the balloon free portion (22) is provided with one or more annular folds (227).

4. The medical endoscope according to claim 1, wherein a peripheral region of the internal opening (241) of the balloon extension tube connected to the balloon free portion (22) is provided with a reinforcing structure, and the reinforcing structure comprises at least one of an annular thick-walled reinforcing rib (223), an upper thickened section (224), and a lower thickened section (225).

5. The medical endoscope according to claim 1, wherein a hollow elastic motive balloon (5) is connected to the external opening of the working channel (112), the elastic motive balloon (5) is compressed to fill a fluid therein into the inner cavity (20) of the balloon to inflate the balloon free portion (22); and after compression is released, the fluid in the inner cavity (20) of the balloon flows back into an inner cavity (50) of the elastic motive balloon.

6. The medical endoscope according to claim 1, wherein a wall thickness and/or strength of the balloon connecting portion (21) of the balloon (2) is greater than that of the balloon free portion (22).

7. A safe-insertion-type endoscope apparatus, comprising a sheath and the medical endoscope according to any one of claims 1-6, wherein wherein an elongated sheath main body (3) is sleeved outside an endoscope main body (1), the sheath main body (3) is provided with a sheath main body internal opening (311) configured to enter a human body during use and a sheath main body external opening (312) configured to be located outside the human body during use; and a flexible balloon shielding portion (23) capable of shielding or covering an outer edge (322) of an internal opening of an inner cavity (31) of the sheath main body extends outward from a junction of a balloon free portion (22) and a balloon connecting portion (21).

8. The safe-insertion-type endoscope apparatus according to claim 7, wherein the flexible balloon shielding portion (23) capable of shielding the outer edge (322) of the internal opening of the inner cavity (31) of the sheath main body extends outward from the junction of the balloon free portion (22) and the balloon connecting portion (21); the flexible balloon shielding portion (23) is provided with a cylindrical flange (232), and the cylindrical flange (232) is configured to cover the outer edge (322) of the internal opening.

9. The safe-insertion-type endoscope apparatus according to claim 7, wherein a fastening mechanism (T) capable of being tightly connected to a tail portion (14) of the endoscope main body is disposed adjacent to a region of the external opening (312) of the sheath main body; during use, a head tip (121) of the endoscope main body (1) protrudes from the internal opening (312) of the sheath main body to expose at least a part of the balloon free portion (22) to the outside; the fastening mechanism (T) is started to tightly connect the sheath main body (3) to the endoscope main body (1), thereby achieving synchronous advancement and retraction of the endoscope main body (1) and the sheath main body (3); and after the apparatus reaches a target position, the fastening mechanism (T) is released, and the endoscope main body (1) is able to rotate freely or move back and forth in the inner cavity (31) of the sheath main body.

10. A transurethral lithotripsy system, comprising the safe-insertion-type endoscope apparatus according to claims 7 to 9, and further comprising at least one of a perfusion apparatus, a lithotripsy laser fiber, a negative pressure suction apparatus, a pressure sensor, a temperature sensor, a stone basket, and a guidewire.
